# EUROPEAN PATENT APPLICATION

(11) **EP 4 610 413 A1**
(43) Date of publication of application: **03.09.2025**
(21) Application number: 23882801.6
(22) Date of filing: 10.04.2023
(51) Int. Cl.: D02J 3/02, D02G 3/44, A61B 17/06, A61B 17/00

(54) **SYSTEM FOR MANUFACTURING LIFTING THREAD HAVING COG FORMED THEREIN AND LIFTING THREAD MANUFACTURED THEREBY**

(30) Priority: 25.10.2022 KR 20220138341
(71) Applicant: JETEMA CO., LTD., Gangwon-do 26355 (KR)
(72) Inventor: LEE, Sang Heon, Daejeon 34067 (KR); KIM, Jae Young, Seoul 05649 (KR); NAM, Jeong Sun, Seoul 04425 (KR); YUN, Bum Jin, Seoul 05502 (KR)
(74) Representative: Brown, Alexander Edward
(86) International application number: PCT/KR2023/004777
(87) International publication number: WO 2024/090691

(57) **Abstract**

An apparatus for manufacturing a lifting thread is provided. **In** one embodiment, the apparatus comprises: a supply part for supplying a yarn; a processing part for manufacturing a lifting thread by forming multidirectional cogs on the surface of the yarn supplied from the supply part; an assembling part that prepares a lifting thread assembly by assembling a tube and a cover with the lifting thread prepared in the processing part; and a packaging part that sterilizes and packages the lifting thread assembly prepared in the assembling part, whereby the efficiency of the process of manufacturing the lifting thread can be improved by continuously performing the entire process from the processing of the yarn to the sterilization packaging of the lifting thread assembly, and the lifting thread having high quality and reliability can be manufactured by performing the entire process under room temperature conditions without applying heat to the yarn.

## Description

### TECHNICAL FIELD

The present invention relates to a system for manufacturing a lifting thread with a cog formed therein and the lifting thread manufactured thereby. Meanwhile, this patent application claims priority to Korean Patent Application No. 10-2022-0138341, filed with the Korean Intellectual Property Office on October 25, 2022, and the disclosure of the patent application is incorporated herein by reference in its entirety.

### BACKGROUND

Generally, a lifting thread is applied in a procedure in which a thread having small cogs, like rose thorns, is inserted into the skin to pull and smooth out wrinkles. A lifting thread is used by implanting a face one thread (or yarn) under the skin to uniformly pull soft tissue, tightening sagging skin and restoring elasticity. In addition to improving wrinkles, it can also address skin laxity and sagging jawlines, making it effective not only for reducing forehead wrinkles and nasolabial folds but also for lifting drooping eyelids, jawlines, and sagging cheeks. Thread lifting is performed using only a special yarn and a needle under simple local anesthesia, which does not strain the body. This reduces the burden on the patient while allowing for immediate effects such as facial lifting, wrinkle removal, and jawline improvement.

The fascia of the human face is a slippery membrane composed of connective tissues and muscles beneath the skin and fat, and it consists of multiple layers rather than a single layer. In addition, the fascia can transmit muscle movements to the skin, playing a role in forming facial expressions, and it is the primary layer where most lifting thread procedures are performed. The retaining ligaments are the tissues that support soft tissues and prevent sagging due to skin aging. As aging progresses and the upper-fat layer descends, the boundaries of these ligaments become more pronounced. In this case, the sagging phenomenon becomes more prominent, and these ligaments can be used as fixation or lifting points in lifting thread procedures. When the skin ages and the fat layer descends, lifting thread procedures can be performed. Thread lifting procedures use lifting threads with cogs, where the projections of the inserted thread engage with tissues such as retaining ligaments and fibrous compounds, physically pulling the superficial muscular aponeurotic system (SMAS) layer.

In this case, the yarn can play a role in supporting tissues by suturing damaged tissues due to surgery or trauma, or as a thread used in plastic surgery. There are two types of yarn: non-absorbable and absorbable yarn. In particular, although the thickness of the yarn does not affect the absorption rate, it is related to tissue response and irritation. It is known that finer yarn heals faster and induces less tissue reaction compared to thicker yarn.

Such a lifting thread is provided with cogs to pull the skin in the desired direction after being inserted into the subcutaneous layer of sagging and drooping skin. Unlike ordinary threads, lifting threads are extremely thin. Therefore, to form fine cogs with uniform height and thickness on such thin suture threads, the molding process must be carried out with high precision, and a highly controlled manufacturing apparatus must be used.

Meanwhile, conventionally, a heat-press molding method has been used to form the cogs on the lifting thread by molding under heating conditions. However, heat treatment of the yarn alters its physical properties or deforms its surface shape, leading to a reliability issue in quality.

In addition, a conventional manufacturing method involved rotating the yarn to form the cogs in multiple directions. However, during the rotation process, the tensile strength and overall durability of the produced lifting thread tended to deteriorate.

In addition, conventional lifting thread manufacturing and packaging processes were carried out intermittently, with separate steps for forming cogs on the yarn, assembling covers or other components onto the lifting thread, and packaging the lifting thread. As a result, work efficiency was low.

### DISCLOSURE

### TECHNICAL PROBLEM

The present invention is directed to providing a lifting thread manufacturing apparatus capable of forming molded cogs on a lifting thread without applying heat to the yarn, allowing the cogs to be formed in multiple directions without rotating the yarn, and continuously performing the processes from supplying the yarn to manufacturing and packaging the lifting thread, as well as a lifting thread manufactured thereby.

### TECHNICAL SOLUTION

To solve the above problems, the present invention provides a lifting thread manufacturing apparatus, including: a supply unit configured to supply a yarn; a processing unit configured to manufacture a lifting thread by forming multidirectional cogs on the surface of the yarn supplied from the supply unit; an assembling unit configured to manufacture a lifting thread assembly by assembling a tube and a cover with the lifting thread manufactured in the processing unit; and a packaging unit configured to sterilize and package the lifting thread assembly manufactured in the assembling unit, wherein the efficiency of the process of manufacturing the lifting thread is improved by continuously performing the entire process from the processing of the yarn to the sterilization and packaging of the lifting thread assembly, and the lifting thread having high quality and reliability is manufactured by performing the entire process under room temperature conditions without applying heat to the yarn.

### ADVANTAGEOUS EFFECT

According to an exemplary embodiment of the present invention, even when forming molded cogs, the compression-cutting-conveying method of the processing unit is controlled so that the process is performed under room temperature conditions without applying heat to the yarn, thereby enabling the manufacture of a lifting thread with high reliability in quality.

In addition, according to an exemplary embodiment of the present invention, by adopting a structure in which the processing unit rotates, cogs can be formed in multiple directions without the need to rotate the raw yarn, thereby enabling the manufacture of a lifting thread that maintains the durability of the yarn, such as tensile strength.

In addition, according to an exemplary embodiment of the present invention, an apparatus with a structure that enables the continuous execution of processes from supplying the yarn to manufacturing and packaging the lifting thread is applied, thereby significantly improving the efficiency of the lifting thread manufacturing process.

In addition, according to an exemplary embodiment of the present invention, an evaluation of the assembled state is automatically performed before packaging the product, thereby simultaneously improving reliability in quality and efficiency in the manufacturing process.

### BRIEF DESCRIPTION OF THE DRAWINGS

FIG. 1 is a schematic diagram illustrating a lifting thread manufacturing apparatus according to an exemplary embodiment of the present invention.
FIG. 2 is a schematic diagram illustrating a processing unit according to an exemplary embodiment of the present invention.
FIG. 3 shows the structure of a processing unit according to an exemplary embodiment of the present invention.
FIG. 4 shows the structure of a processing unit according to another exemplary embodiment of the present invention.
FIG. 5 is a schematic diagram illustrating an assembling unit and a packaging unit in a lifting thread manufacturing apparatus according to an exemplary embodiment of the present invention.
FIGS. 6 to 10 illustrate the steps of assembling a lifting thread in an assembling unit according to an exemplary embodiment of the present invention.
FIGS. 11 and 12 illustrate the inspection of the assembled state of a lifting thread by an inspection unit according to an exemplary embodiment of the present invention.
FIGS. 13 to 16 illustrate the steps of packaging a lifting thread assembly in a packaging unit according to an exemplary embodiment of the present invention.
FIGS. 17 to 24 illustrate the lifting thread manufactured by a lifting thread manufacturing apparatus according to an exemplary embodiment of the present invention.
FIG. 25 illustrates a cover according to an exemplary embodiment of the present invention.

### MODES OF THE INVENTION

Hereinafter, embodiments of the present invention will be described in detail. However, the present invention is not limited to the embodiments disclosed below and may be implemented in various different forms. The following embodiments are merely provided to ensure a complete disclosure of the present invention and to fully convey the scope of the invention to those skilled in the art.

In the present specification, when an element is described as being positioned "above" or "below" another element, this includes both cases where the element is directly above or below the other element and cases where additional elements are interposed between them. In the present specification, the terms "upper" and "lower" are relative concepts defined based on the observer's viewpoint. Depending on the observer's perspective, "upper" may refer to "lower," and "lower" may refer to "upper."

In multiple figures, the same reference numerals denote substantially identical elements. It should be understood that the terms "comprise or include" or "have" or the like when used in this specification, are intended to specify the presence of stated features, numbers, steps, operations, elements, components and/or a combination thereof but not preclude the possibility of the presence or addition of one or more other features, numbers, steps, operations, elements, components, or a combination thereof.

In addition, the term "cutting" as referred to in the present invention is intended for forming cogs on the yarn and includes both molding cutting and simple cutting.

In addition, in the present invention, the term "yarn" refers to the form supplied from the supply unit before cogs are formed, the term "lifting thread" refers to the form in which cogs have been formed on the yarn, and the term "lifting thread assembly" may refer to a state in which a tube is installed on the lifting thread or a state in which both a tube and a cover are installed.

The inventors of the present invention recognized that, in the conventional process of forming cogs on a lifting thread, heat treatment of the yarn could alter its physical properties or deform its surface shape, leading to reduced reliability in the quality of the manufactured lifting thread. Additionally, the conventional method of rotating the yarn to form cogs in multiple directions posed a problem in which the tensile strength and durability of the produced lifting thread deteriorated due to the rotation process. Aiming to solve these issues while enabling a continuous process from cog formation on the yarn to the packaging of the lifting thread, the inventors developed the present invention through extensive trial and error, ultimately devising a lifting thread manufacturing apparatus that addresses these challenges.

Hereinafter, a detailed description will be given with reference to the drawings.

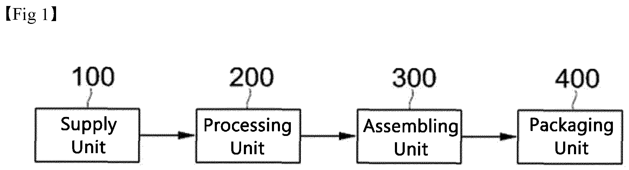

FIG. 1 is a schematic diagram illustrating a lifting thread manufacturing apparatus according to an exemplary embodiment of the present invention.

Referring to FIG. 1, the lifting thread manufacturing apparatus includes a supply unit 100 that supplies yarn, a processing unit 200 that forms multidirectional cogs on the surface of the yarn supplied from the supply unit 100 to manufacture a lifting thread, an assembling unit 300 that assembles a tube and a cover onto the lifting thread manufactured in the processing unit 200 to produce a lifting thread assembly, and a packaging unit 400 that sterilizes and packages the lifting thread assembly manufactured in the assembling unit 300.

The arrows shown in FIG. 1 indicate the movement direction of the yarn. The present invention enables the entire process, from the supply of the yarn to packaging, to be performed and inspected automatically without operator intervention.

According to the lifting thread manufacturing apparatus of the present invention, the entire process, from the processing of the yarn to the sterilization and packaging of the lifting thread assembly, is performed continuously, improving the efficiency of the lifting thread manufacturing process. Additionally, since the entire process is carried out under room temperature conditions without applying heat to the yarn, a lifting thread with high reliability in quality can be manufactured.

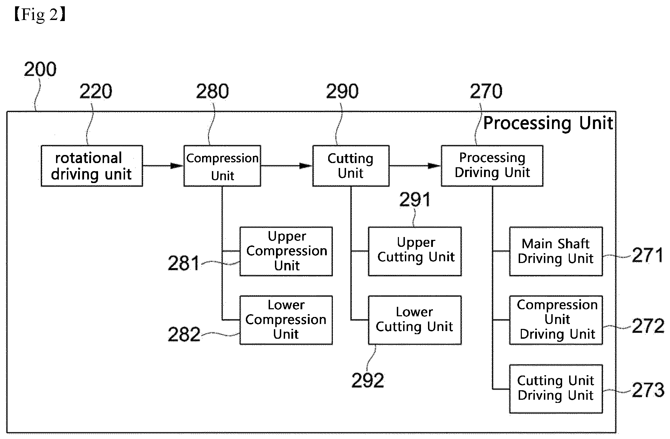

FIG. 2 is a schematic diagram illustrating a processing unit according to an exemplary embodiment of the present invention.

Referring to FIG. 2, the processing unit 200 includes a compression unit 280 that compresses the yarn, a cutting unit 290 that forms cogs on the yarn, a processing driving unit 270 that controls the operation of the compression unit 280 and the cutting unit 290, and a rotational driving unit 220 that rotates the compression unit 280, the cutting unit 290, and the processing driving unit 270.

In the processing unit 200 of the lifting thread manufacturing apparatus according to the present invention, the rotational driving unit 220 is applied to form cogs in multiple directions without rotating the yarn, thereby maintaining the durability of the yarn.

The compression unit 280 and the cutting unit 290 are sequentially arranged in the movement direction of the yarn. For example, the compression unit 280 and the cutting unit 290 may each include two or more units based on the yarn. For example, the compression unit 280 includes an upper compression unit 281 and a lower compression unit 282, and at least one of the upper compression unit 281 and the lower compression unit 282 may move in a vertical direction relative to the movement direction of the yarn. For example, the cutting unit 290 includes an upper cutting unit 291 and a lower cutting unit 292, and at least one of the upper cutting unit 291 and the lower cutting unit 292 may move in a vertical direction relative to the movement direction of the yarn.

The processing driving unit 270 may include a main shaft driving unit 271, a compression unit driving unit 272, and a cutting unit driving unit 273, wherein the compression unit driving unit 272 and the cutting unit driving unit 273 are controlled according to the driving method of the main shaft driving unit 271 to ensure that the four operations-yarn movement, compression, cutting, and rotation-are performed in a predetermined sequence in the processing unit 200. For example, the processing driving unit 270 may include a piston.

In the processing unit 200, the four operations-yarn movement, compression, cutting, and rotation-may be controlled to form cogs in multiple directions on the yarn.

For example, the four operations may include: the yarn moving to the compression unit 280; the compression unit 280 compressing a predetermined area (not shown) of the yarn and then releasing the compression; the released predetermined area (not shown) of the yarn moving to the cutting unit 290; the cutting unit 290 cutting the predetermined area (not shown) of the yarn; the compression unit 280 and the cutting unit 290 rotating by the driving of the rotational driving unit 220; the compression unit 280 compressing and then releasing the rear area (not shown) of the predetermined area (not shown); the released rear end area (not shown) of the yarn moving to the cutting unit 290; the cutting unit 290 cutting the rear end area (not shown) of the yarn; and the compression unit 280 and the cutting unit 290 rotating by the driving of the rotational driving unit 220. For example, the above-described process may be performed in the stated sequence or simultaneously.

For example, the compression may be performed under processing conditions of a thickness of 0.2 to 0.5 mm and within 10 seconds, depending on the thickness of the lifting thread 5, and after the compression is released, the cutting operation may be performed to form a shape with a width of 0.2 to 1.1 mm, depending on the thickness of the lifting thread.

The present invention enables continuous processing by performing the cutting process after the compression is released, rather than cutting simultaneously with compression. This allows for effective shaping according to the desired molding conditions, thereby improving the reliability of the product quality.

**In** addition, by controlling the four operations of "yarn movement, compression, cutting, rotation," the present invention enables the processing to be performed at room temperature without applying heat to the yarn. As a result, not only simple cogs but also molded cogs can be formed while maintaining the reliability of the yarn quality.

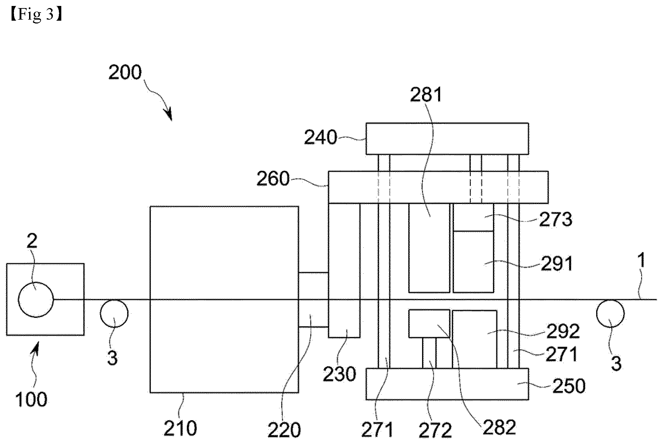

FIG. 3 shows the structure of a processing unit according to an exemplary embodiment of the present invention.

Referring to FIG. 3, the processing unit 200 may include a support unit 210, a rotational driving unit 220, a side support plate 230, an upper support plate 240, a lower support plate 250, a fixing plate 260, a main shaft driving unit 271, a compression unit driving unit 272, a cutting unit driving unit 273, an upper compression unit 281, a lower compression unit 282, an upper cutting unit 291, and a lower cutting unit 292. The aforementioned effects of the present invention can be achieved through the combination and operation of these components.

The support unit 210 is provided to support the rotational motion of the processing unit 200 while not interfering with the movement of the yarn 1, which is wound around the bobbin 2 of the supply unit 100 and conveyed by the yarn conveying means 3. For example, a through-hole (not shown) through which the yarn 1 passes may be formed in the support unit 210, and an additional yarn conveying means 3 may be provided in the through-hole (not shown) so that the yarn 1 is conveyed with appropriate tension. For example, the support unit 210 may have a fixed structure in the processing unit 200.

The rotational driving unit 220 is connected to the support unit 210 and capable of rotational motion, and may include, for example, driving by a motor. For example, a through-hole (not shown) through which the yarn 1 passes may be formed in the rotational driving unit 220. The rotational driving unit 220 can rotate at a preset angle and, for example, can rotate to +30°, +60°, -30°, and -60° when the initial position is set to 0°. Accordingly, it can form helical multi-directional cogs in a total of six directions.

The side support plate 230 may be connected to the rotational driving unit 220 and rotate by the rotational motion of the rotational driving unit 220. For example, a through-hole (not shown) through which the yarn 1 passes may be formed in the rotational driving unit 220.

The fixing plate 260 may extend in the movement direction of the yarn 1 from one end of the side support plate 230 and rotate by the rotational motion of the side support plate 230.

The upper support plate 240 and the lower support plate 250 may be arranged to face each other with the fixing plate 260 therebetween and may be interconnected by at least one main shaft driving unit 271. For example, to improve space utilization efficiency, the main shaft driving unit 271 may have a structure that penetrates the fixing plate 260.

Between the upper support plate 240 and the lower support plate 250, the compression unit 280 and the cutting unit 290, as previously described with reference to FIG. 2, may be sequentially arranged in the movement direction of the yarn 1.

The upper compression unit 281 may be fixed to the fixing plate 260, and the lower compression unit 282 may be connected to the lower support plate 250 and the compression unit driving unit 272.

The upper cutting unit 291 may be connected to the upper support plate 240 and the cutting unit driving unit 273, and the lower cutting unit 292 may be fixed by being connected to the fixing plate 260 through a separate connection member (not shown).

The structure constituting the aforementioned processing unit 200 may have some components that can move in the vertical direction relative to the movement direction of the yarn by the main shaft driving unit 271, the compression unit driving unit 272, and the cutting unit driving unit 273.

For example, the fixing plate 260, the upper compression unit 281, and the lower cutting unit 292 may be fixed, while the upper support plate 240, the lower support plate 250, the lower compression unit 282, and the upper cutting unit 291 may move in the vertical direction relative to the movement direction of the yarn 1. For example, the upper support plate 240 and the lower support plate 250 may move together by the driving of the main shaft driving unit 271. For example, a conventional driving member (not shown) that imparts a driving force to move the upper support plate 240 and the lower support plate 250 in the vertical direction relative to the movement direction of the yarn 1 may be provided on either the upper support plate 240 or the lower support plate 250. For example, the conventional driving member may be provided on the side of the driving unit 200 or on the upper side of the upper support plate 240. The driving force generated by the conventional driving member may be transmitted to the upper support plate 240 and the lower support plate 250, as well as to the compression unit 280 and the cutting unit 290, which are connected to them via the processing driving unit 270, thereby controlling the compression-cutting operation.

For example, as the lower support plate 250 moves toward the upper support plate 240 by the driving of the main shaft driving unit 271, the lower compression unit 282 and the upper compression unit 281 may compress the yarn 1 positioned between them. Thereafter, as the upper support plate 240 moves toward the lower support plate 250 by the driving of the main shaft driving unit 271, the compressive force applied to the yarn 1 may be released. Thereafter, the yarn 1 may be moved by the yarn conveying means 3 so that the previously compressed area is positioned between the upper cutting unit 291 and the lower cutting unit 292. Thereafter, as the upper support plate 240 moves toward the lower support plate 250 by the driving of the main shaft driving unit 271, the upper cutting unit 291 and the lower cutting unit 292 may cut the yarn 1 positioned between them. Thereafter, as the lower support plate 250 moves toward the upper support plate 240 by the driving of the main shaft driving unit 271, the upper cutting unit 291 may be separated from the lower cutting unit 292. Thereafter, the operation of moving the yarn 1 by the yarn conveying means 3 and the operation of rotating the compression unit 280, the cutting unit 290, and the processing driving unit 270 around the yarn 1 by the driving of the rotational driving unit 220 may be performed.

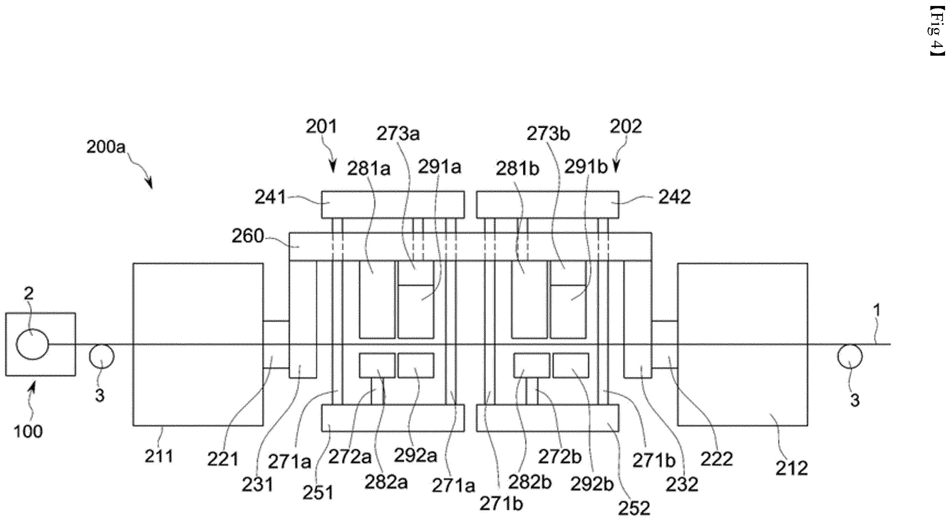

FIG. 4 shows the structure of a processing unit according to another exemplary embodiment of the present invention.

**In** the present invention, the processing unit 200a may include at least one pair of compression units and cutting units, at least one pair of processing driving units, and at least one pair of rotational driving units that rotate the compression units and the cutting units. Although FIG. 4 illustrates an example including one pair of the processing units shown in FIG. 3, it is also possible to include more than one of the processing unit structures shown in FIG. 3 between the pair of support units and the rotational driving units. The names and arrangement of each structure in FIG. 4 may be applied in the same manner as described with reference to FIG. 3.

For example, the processing unit 200a may include a first support unit 211 and a second support unit 212 forming both end portions, with a first rotational driving unit 221 connected to the first support unit 211 and a second rotational driving unit 222 connected to the second support unit 212.

The first rotational driving unit 221 may be connected to a first side support plate 231, and the second rotational driving unit 222 may be connected to a second side support plate 232.

In FIG. 4, the first side support plate 231 and the second side support plate 232 are depicted as being connected to a single fixing plate 260. However, each of the first side support plate 231 and the second side support plate 232 may be connected to different fixing plates, allowing each fixing plate to rotate according to the rotation of the respective first side support plate 231 and second side support plate 232.

In the processing unit 200a shown in FIG. 4, the structure arranged at the front end may include a first upper support plate 241, a first lower support plate 251, a first main shaft driving unit 271a, a first compression unit driving unit 272a, a first cutting unit driving unit 273a, a first upper compression unit 281a, a first lower compression unit 282a, a first upper cutting unit 291a, and a first lower cutting unit 292a. The structure arranged at the rear end may include a second upper support plate 242, a second lower support plate 252, a second main shaft driving unit 271b, a second compression unit driving unit 272b, a second cutting unit driving unit 273b, a second upper compression unit 281b, a second lower compression unit 282b, a second upper cutting unit 291b, and a second lower cutting unit 292b. Although not shown in FIG. 4, the structure arranged at the n^{th} position of 3 or more may include an n^{th} upper support plate, an n^{th} lower support plate, an n^{th} main shaft driving unit, an n^{th} compression unit driving unit, an n^{th} cutting unit driving unit, an n^{th} upper compression unit, an n^{th} lower compression unit, an n^{th} upper cutting unit, and an n^{th} lower cutting unit.

The specific arrangement and driving method of each structure at the front end and the rear end may be applied as described above with reference to FIG. 3.

For example, according to the structure of the processing unit 200a in FIG. 4, at least one pair of the compression units and the cutting units may simultaneously form cogs in a region spaced a predetermined distance from the yarn 1.

For example, the shape of the cogs formed by the first upper cutting unit 291a and the first lower cutting unit 292a may be the same as or different from the shape of the cogs formed by the second upper cutting unit 291b and the second lower cutting unit 292b, and the formation angles of the cogs may be the same or different.

For example, the cogs formed by the first upper cutting unit 291a and the first lower cutting unit 292a and the cogs formed by the second upper cutting unit 291b and the second lower cutting unit 292b may have the same shape but be formed with inclination angles in opposite directions.

Meanwhile, after being supplied from the supply unit 100 to the processing unit 200, 200a, where the lifting thread is manufactured, it may be cut into preset length segments and then conveyed to the assembling unit as cut segments. For example, the cut segments may be conveyed along a conveyor, gripped and conveyed by a jig, or conveyed through a combination of these methods.

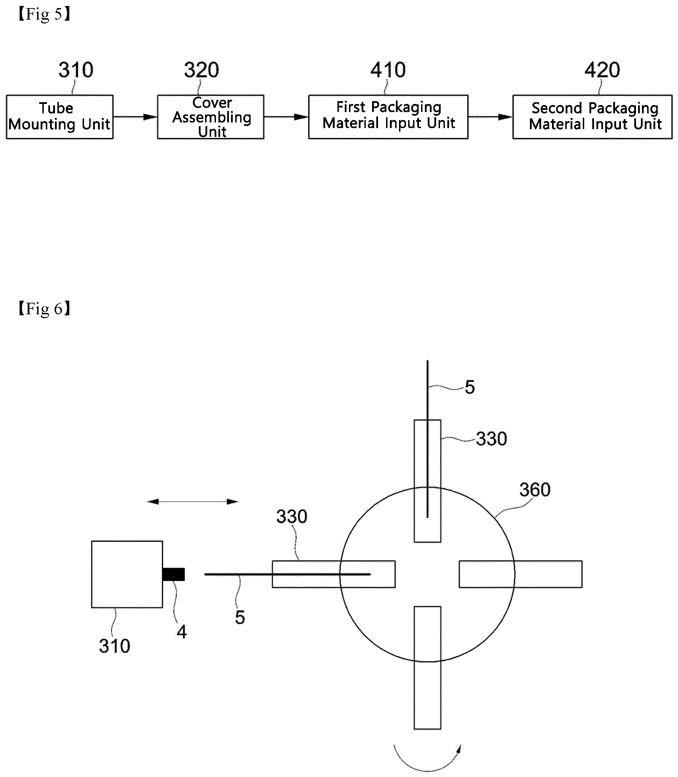

FIG. 5 is a schematic diagram illustrating an assembling unit and a packaging unit in a lifting thread manufacturing apparatus according to an exemplary embodiment of the present invention.

Referring to FIG. 5, the assembling unit 300 includes a tube mounting unit 310 and a cover assembling unit 320, and the packaging unit 400 includes a first packaging material input unit 410 and a second packaging material input unit 420. **In** FIG. 5, the arrows indicate the movement direction of the lifting thread, and the process from assembly to packaging may be continuously performed and inspected without operator intervention.

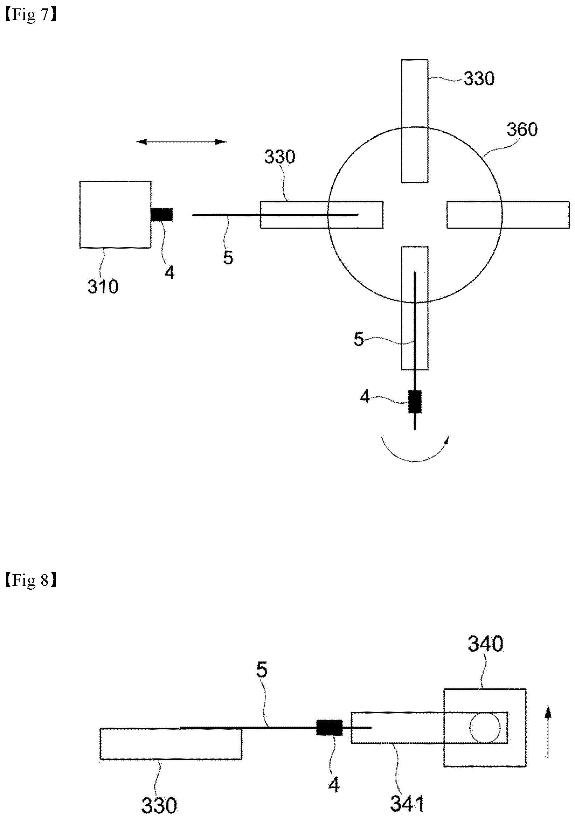

The tube mounting unit 310 installs a tube at one end of the lifting thread, and the cover assembling unit 320 assembles a cover onto the lifting thread with the installed tube to form a lifting thread assembly, which will be described in detail with reference to FIGS. 6 and 7.

FIGS. 6 to 10 illustrate the steps of assembling a lifting thread in an assembling unit according to an exemplary embodiment of the present invention.

Referring to FIGS. 6 and 7, the assembling unit 300 may include a rotating plate 360, and referring to Figs. 8 to 10, the assembling unit 300 may include an assembling unit conveying unit 340.

The rotating plate 360 is provided with a plurality of lifting thread seating units 330, on which the lifting thread 5 is seated, and is capable of rotational movement. The lifting thread 5, which is processed from the yarn (1 in FIG. 3) in the processing unit (100 in FIG. 3), may be conveyed and seated on the lifting thread seating unit 330.

The assembling unit conveying unit 340 may grip the lifting thread 5 seated on the lifting thread seating unit 330, detach it from the rotating plate 360, and convey it to the packaging unit (400 in FIG. 1).

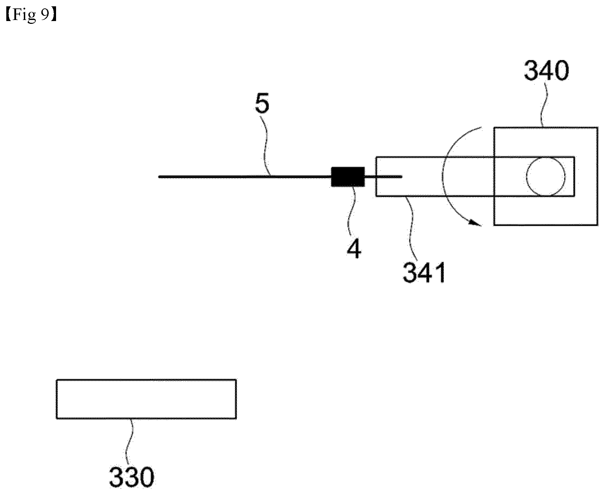

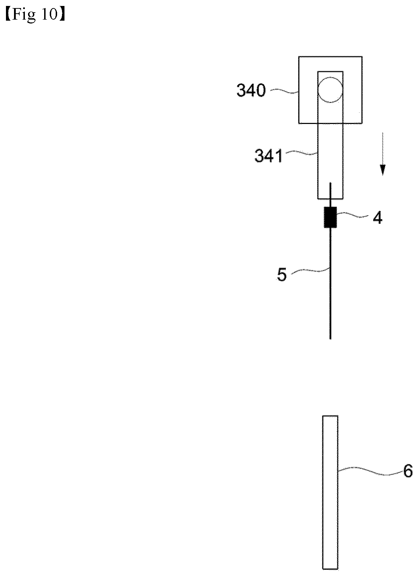

After the lifting thread 5 processed in the processing unit (200 in FIG. 1) is seated on the lifting thread seating unit 330, the tube mounting unit 310 may mount a tube 4 onto one lifting thread 5 seated on one lifting thread seating unit 330, as shown in FIG. 6. For example, when the lifting thread seating unit 330 is positioned at a predetermined location by the rotation of the rotating plate 360, the tube mounting unit 310 may perform a reciprocating motion to mount the tube 4 and return to its original position. After the tube 4 is mounted on the lifting thread 5, the rotating plate 360 may rotate by a predetermined angle in the direction indicated by the arrows. Then, as shown in FIG. 7, the tube mounting unit 310 may mount a tube 4 onto another lifting thread 5 seated behind the previous lifting thread 5 in the rotation direction of the rotating plate 360. Simultaneously or sequentially, as shown in FIG. 8, the assembling unit conveying unit 340 may grip the lifting thread 5 with the mounted tube 4 and detach it from the lifting thread seating unit 330, as shown in FIG. 9. For example, an assembling unit conveying unit jig 341 mounted in the assembling unit conveying unit 340 may grip the lifting thread 5. Thereafter, as shown in FIG. 10, the movement of the assembling unit conveying unit 340 may allow the lifting thread 5 to be assembled with the cover 6.

For example, the tube 4 may function to secure the lifting thread 5 to a needle and may be formed of a styrofoam material.

For example, in FIG. 8, after the assembling unit conveying unit jig 341 grips the lifting thread 5, the assembling unit conveying unit 340 may move upward in the vertical direction relative to the rotating plate 360 to the lifting thread seating unit 330. Then, in FIG. 9, the assembling unit conveying unit jig 341 may rotate 90° relative to the assembling unit conveying unit 340. **In** FIG. 10, the assembling unit conveying unit 340 may move downward in the vertical direction relative to the rotating plate 360 to the lifting thread seating unit 330, allowing the lifting thread 5 to be assembled with the cover 6. By this method, the efficiency of space utilization in the processing area can be improved.

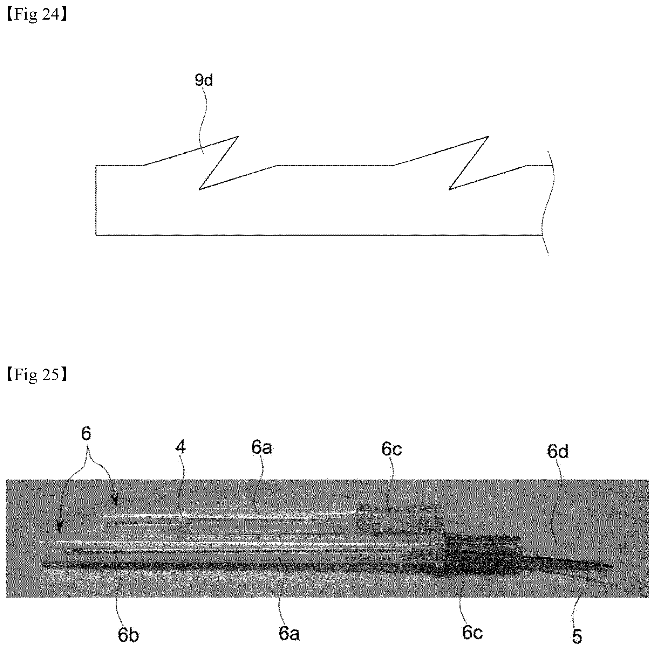

Referring to FIG. 25, the cover 6 may include a needle cap 6a, a catheter cannula 6b, a hub 6c, and a stopper 6d.

The needle cap 6a may function as a cover to protect the tip of the catheter cannula 6b and the lifting thread 5.

The catheter cannula 6b, also referred to as a needle tube, may function as a needle for inserting the absorbable lifting thread 5 into the tissue (skin) as the part inserted into the body.

The hub 6c may function as a handle during the procedure.

The stopper 6d may function to secure the lifting thread 5 to the hub 6c.

For example, the assembling unit conveying unit 340 may descend in the vertical direction relative to the rotating plate 360 to the lifting thread seating unit 330 to insert the lifting thread 5 into the assembly of the catheter cannula, the hub, and the stopper, and then assemble it with the needle cap to manufacture the lifting thread assembly 5a.

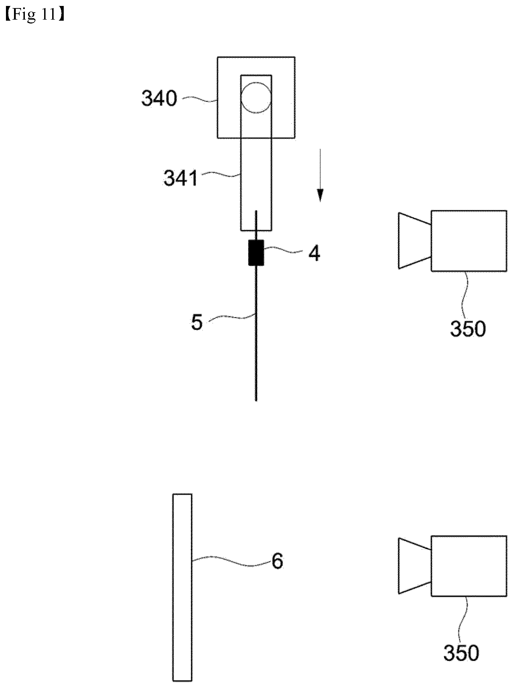

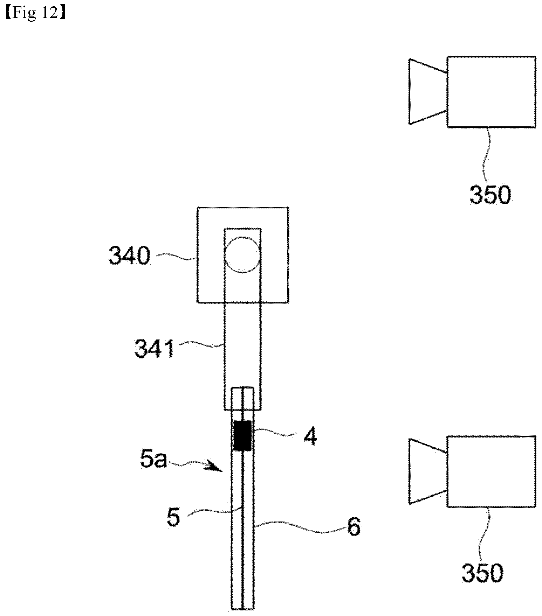

FIGS. 11 and 12 illustrate the inspection of the assembled state of a lifting thread by an inspection unit according to an exemplary embodiment of the present invention.

The inspection unit 350 may perform at least one of inspecting the installation state of the lifting thread 5 and the tube 4, as shown in FIG. 11, and inspecting the assembly state of the lifting thread assembly 5a, as shown in FIG. 12.

For example, as described with reference to FIG. 9, after the assembling unit conveying unit jig 341 rotates 90° relative to the assembling unit conveying unit 340, the inspection unit 350 may perform a first inspection of the installation state of the lifting thread 5 and the tube 4, as shown in FIG. 11. For example, based on the image information captured by the inspection unit 350, whether there is an abnormality in the installation state may be determined, and the result may be displayed on a display. If an abnormality is detected, the corresponding lifting thread 5 may be conveyed to a separately provided accumulation unit (not shown) instead of proceeding to the assembling step.

For example, after the inspection by the inspection unit 350 is performed as shown in FIG. 11, the assembling unit conveying unit 340 may first descend, allowing the inspection unit 350 to perform a second inspection to determine whether there is any abnormality in the region opposite to where the tube 4 is mounted on the lifting thread 5. Thereafter, the assembling unit conveying unit 340 may ascend again, move to the location where the cover 6 is placed, and then descend for the second time to assemble the cover 6 onto the lifting thread 5. Thereafter, the assembling unit conveying unit 340 may ascend again, move to the location where the inspection unit 350 is placed, and then descend for the third time to verify the assembled state of the cover 6 and the lifting thread 5 for the third inspection. For example, during the third inspection, the assembly state of the needle cap and the catheter cannula, as well as the assembly state of the stopper, may be inspected. For example, at least one of the first to third inspections may include a vision inspection.

Thus, the present invention can significantly enhance the reliability of quality by inspecting the quality of the lifting thread assembly 5a through three stages of inspection.

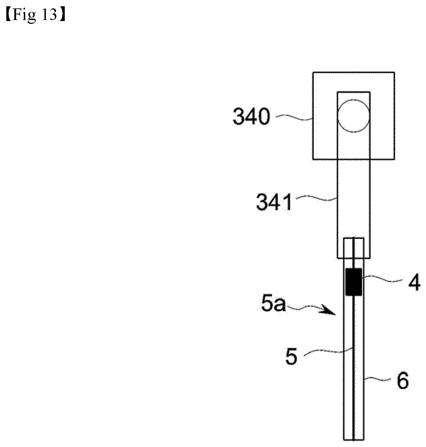

FIGS. 13 to 16 illustrate the steps of packaging a lifting thread assembly in a packaging unit according to an exemplary embodiment of the present invention.

Referring to FIG. 5 and FIGS. 13 to 16, the packaging unit (400 in FIG. 1) includes a first packaging material input unit 410 and a second packaging material input unit 420.

Referring to FIG. 13, the lifting thread assembly 5a that has been inspected by the inspection unit (350 in FIGS. 11 and 12), as shown in FIG. 12, may be prepared.

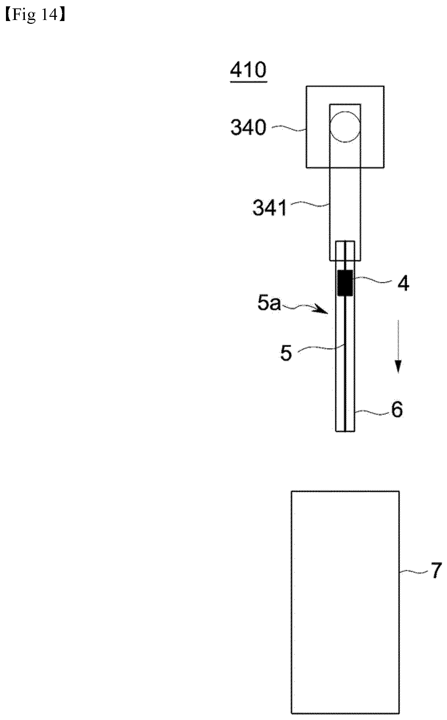

Referring to FIG. 14, the assembling unit conveying unit 340 may insert the lifting thread assembly 5a into a first packaging material 7 at the first packaging material input unit 410.

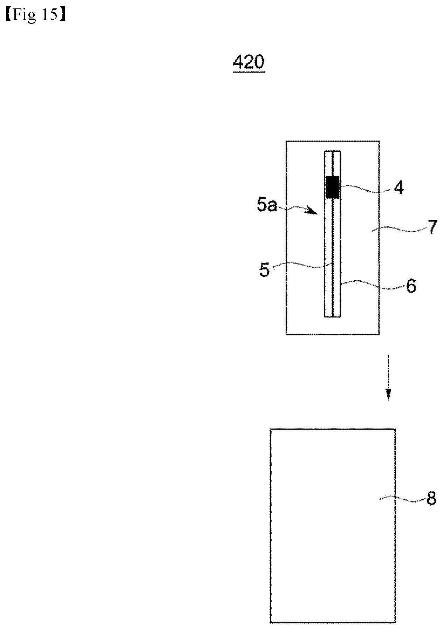

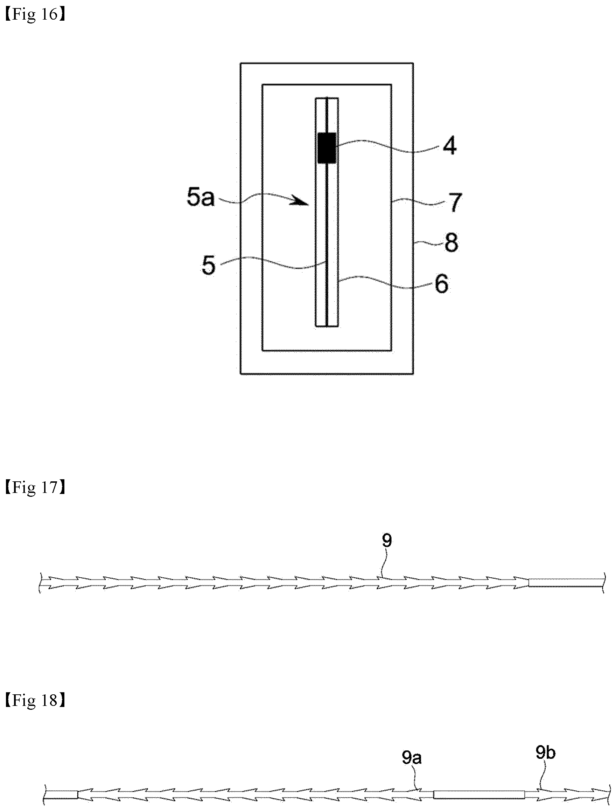

Referring to FIGS. 15 and 16, the first packaging material 7 containing the lifting thread assembly 5a may be inserted into a second packaging material 8 at the second packaging material input unit 420.

For example, the first packaging material input unit 410 and the second packaging material input unit 420 may be spatially separated. For example, the second packaging material input unit 420 may be positioned below the first packaging material input unit 410, allowing the lifting thread assembly 5a to be sequentially inserted into the first packaging material 7 and then into the second packaging material 8 without requiring a separate jig, thereby improving space utilization efficiency.

For example, the first packaging material 7 may include an aluminum pouch, and the second packaging material 8 may include sterilization paper.

For example, the packaging process in the packaging unit (400 in FIG. 1) may include attaching a label to the first packaging material 7, inserting one or more lifting thread assemblies 5a into the opening of the first packaging material 7, sealing the opening of the first packaging material 7, inserting the first packaging material 7 into the opening of the second packaging material 8, sealing the opening of the second packaging material 8, and discharging the second packaging material 8 to a storage unit (not shown). In this case, the packaging process may further include sterilizing the sealed first packaging material 7 with EO gas before it is inserted into the second packaging material 8. In addition, inserting the first packaging material 7 into the opening of the second packaging material 8 and sealing the opening of the second packaging material 8 may be performed inside a glove box.

FIGS. 17 to 24 illustrate the lifting thread manufactured by a lifting thread manufacturing apparatus according to an exemplary embodiment of the present invention.

As described above, in the present invention, both the molding cutting and the simple cutting may be performed in the processing unit, wherein embodiments of the molding cutting are shown in FIGS. 17 to 23, and an embodiment of the simple cutting is shown in FIG. 24.

In the present invention, as described with reference to FIG. 3, the processing unit can control three types of movements-yarn movement, compression, and cutting-to form a one-dimensional cog in a planar shape, and control four types of movements-yarn movement, compression, cutting, and rotation-to form cogs in two-dimensional, three-dimensional, or more complex directions.

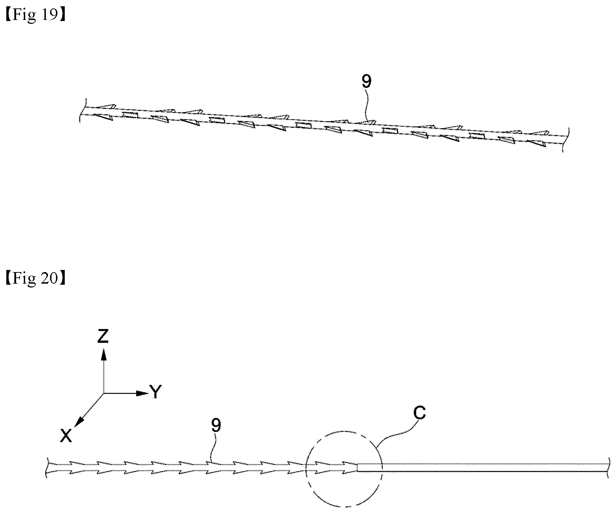

FIG. 17 illustrates the shape of the lifting thread manufactured by the processing unit described above with reference to FIG. 3, FIG. 18 illustrates the shape of a lifting thread in which the cogs (9a, 9b) have the same shape but are formed with opposite inclination angles, as described above with reference to FIG. 4, and FIG. 19 illustrates a lifting thread manufactured according to an exemplary embodiment of the present invention. Referring to FIG. 19, it can be seen that, in the lifting thread according to an exemplary embodiment of the present invention, the cogs 9 are formed in a helical multi-directional manner on the yarn.

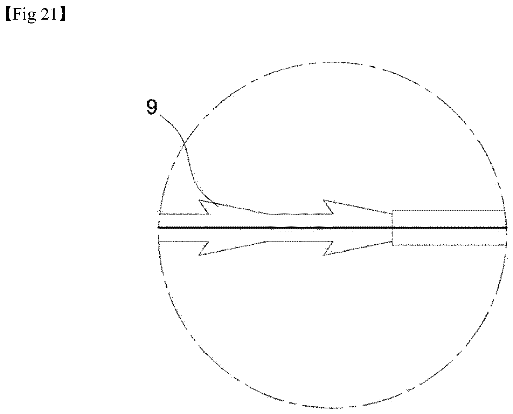

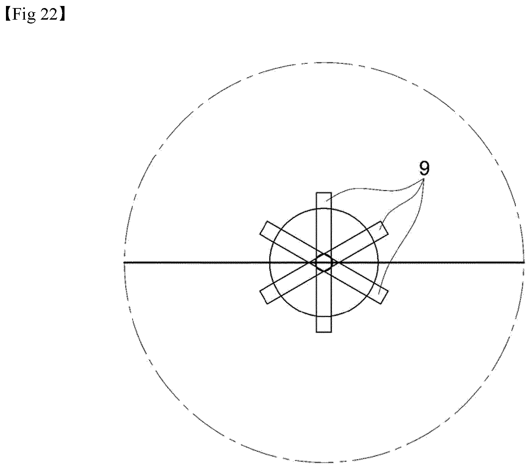

FIGS. 20 to 22 illustrate that the cogs 9 are formed in a three-dimensional direction by controlling the four motion operations of 'yarn movement-compression-cutting-rotation' in the processing unit described with reference to FIG. 3. Specifically, FIG. 21 is an enlarged view of the region (c) indicated in FIG. 20, and FIG. 22 shows FIG. 20 as viewed in the longitudinal direction of the lifting thread, i.e., the y-direction. Referring to FIGS. 19 and 22, it can be seen that the lifting thread manufactured according to an exemplary embodiment of the present invention has cogs 9 uniformly formed in a consistent direction.

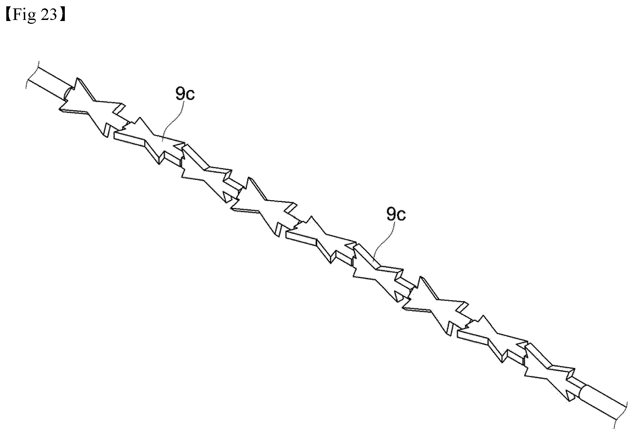

FIG. 23 illustrates that the cogs 9c are formed in a two-dimensional direction by controlling the four motion actions of 'yarn movement - compression - cutting - rotation' in the aforementioned processing unit with reference to Fig. 3. It shows that, unlike the barbed-shaped cogs of the lifting thread according to FIGS. 17 to 22, a butterfly-shaped cogs 9c are formed.

FIG. 24 illustrates the cogs 9d manufactured by performing the aforementioned simple cutting. For example, it may be formed by incising the yarn to a predetermined depth at a predetermined angle from the side of the yarn in the aforementioned cutting unit.

Meanwhile, according to an exemplary embodiment of the present invention, the yarn forming the cogs may be made of a material having elasticity.

For example, the yarn may be made of a bioabsorbable medical polymer material, polypropylene, nylon, or a bio-nonabsorbable medical polymer material composed of any one selected from a mixture thereof. For example, the yarn may be made of a polydioxanone filament with a specification of USP 2 to USP 5-0.

For example, the bioabsorbable medical polymer material may include any one selected from polydioxanone (PDO), poly(L-lactic acid) (PLLA), polyglycolic acid (PGA), polycaprolactone (PCL), and copolymers formed from combinations thereof.

Polydioxanone (PDO) undergoes hydrolysis and is excreted in the urine. During degradation, it stimulates collagen production, promoting skin regeneration and improving skin elasticity. It is sensitive to heat and may degrade over a period of 6 to 8 months.

Poly(L-lactic acid) (PLLA), which is used as a raw material for Sculptra, begins to degrade into H₂O, CO₂, and glucose approximately two months after administration. Through degradation, it can continuously stimulate collagen production. Threads made of poly(L-lactic acid) (PLLA) have minimal elasticity, making them hard and stiff, which may cause a foreign body sensation and pain after insertion. They can last for up to 18 months.

Polycaprolactone (PCL), which is used as a raw material for Ellansé, degrades into H₂O and CO₂ and can continuously stimulate collagen production through its degradation process. Threads made of polycaprolactone (PCL) are flexible and soft, causing minimal foreign body sensation. However, due to their low elasticity, they are difficult to shape. Polycaprolactone (PCL) can last for up to 24 months.

For example, the lifting thread may be made of various forms of PDO yarn, such as cutting threads, bidirectional cog threads (2D), Epiticon, N-Fix, Concertina, Scaffold, and TESSLIFT-SOFT (including cutting cog core threads and mesh).

As described above, the detailed description of the present invention has been provided based on exemplary embodiments with reference to the accompanying drawings. However, the above embodiments are merely preferred examples for explaining the present invention and should not be construed as limiting the invention thereto. The scope of the present invention should be understood based on the claims set forth below and their equivalents.

For example, the drawings schematically illustrate each component as the main subject to aid understanding. The thickness, length, number, and other aspects of the illustrated components may differ from actual specifications due to the nature of the drawing process. In addition, the material, shape, and dimensions of each component presented in the above embodiments are merely examples and are not particularly limited. Various modifications can be made without substantially departing from the effects of the present invention.

**Description of Symbols**

| | | | |
|---|---|---|---|
| 1: yarn 2: bobbin | 3: yarn conveying means | 4: tube | |
| 5: lifting thread | 5a: lifting thread assembly | 6: cover | 6a: needle cap |
| 6b: catheter cannula | 6c: hub6d: stopper | 7: first packaging material | |
| 8: second packaging material | 9, 9a, 9b, 9c, 9d: cog | 100: supply unit | |
| 200, 200a: processing unit | 300: assembling unit | 400: packaging unit | |
| 101: bobbin | | | |
| 201: first processing unit | 202: second processing unit | 210: support unit | |
| 211: first support unit | | | |
| 212: second support unit | 220: rotational driving unit | 221: first rotational driving unit | |
| 222: second rotational driving unit | 230: side support plate | 231: first side support plate | |
| 232: second side support plate | 240: upper support plate | 241: first upper support plate | |
| 242: second upper support plate | 250: lower support plate | 251: first lower support plate | |
| 252: second lower support plate | 260: fixing plate | 270: processing driving unit | |
| 271: main shaft driving unit | 271a: first main shaft driving unit | 271b: second main shaft driving unit | |
| 272: compression unit driving unit | 272a: first compression unit driving unit | | |
| 272b: second compression unit driving unit | | | |
| 273: cutting unit driving unit | 273a: first cutting unit driving unit | 273b: second cutting unit driving unit | |
| 280: compression unit | 281: upper compression unit | 281a: first upper compression unit | |
| 281b: second upper compression unit | 282: lower compression unit | 282a: first lower compression unit | |
| 282b: second lower compression unit | 290: cutting unit | 291: upper cutting unit | |
| 291a: first upper cutting unit | 291b: second upper cutting unit | 292: lower cutting unit | |
| 292a: first lower cutting unit | 292b: second lower cutting unit | 310: tube mounting unit | |
| 320: cover assembling unit | 330: lifting thread seating unit | 340: assembling unit conveying unit | |
| 341: assembling unit conveying unit jig | 350: inspection unit | 360: rotating plate | |
| 410: first packaging material input unit | 420: second packaging material input unit | | |

## Claims

1. A lifting thread manufacturing apparatus, comprising:
a supply unit configured to supply a yarn;
a processing unit configured to manufacture a lifting thread by forming multidirectional cogs on the surface of the yarn supplied from the supply unit;
an assembling unit configured to manufacture a lifting thread assembly by assembling a tube and a cover with the lifting thread manufactured in the processing unit; and
a packaging unit configured to sterilize and package the lifting thread assembly manufactured in the assembling unit,
wherein the efficiency of the process of manufacturing the lifting thread is improved by continuously performing the entire process from the processing of the yarn to the sterilization and packaging of the lifting thread assembly, and the lifting thread having high quality and reliability is manufactured by performing the entire process under room temperature conditions without applying heat to the yarn, and
wherein the processing unit comprises a rotational driving unit, thereby maintaining the durability of the yarn by forming the cogs in multiple directions without rotating the yarn.

2. The lifting thread manufacturing apparatus of claim 1,
wherein the processing unit comprises:
a compression unit configured to compress the yarn;
a cutting unit configured to form the cogs on the yarn;
a processing driving unit configured to control the operation of the compression unit and the cutting unit; and
a rotational driving unit configured to rotate the compression unit, the cutting unit, and the processing driving unit.

3. The lifting thread manufacturing apparatus of claim 2,
wherein the processing unit sequentially arranges the compression unit and the cutting unit in the moving direction of the yarn, and to form the cogs in multiple directions, the processing unit is configured to:
move the yarn to the compression unit;
compress a predetermined area of the yarn with the compression unit and then release the compression;
move the predetermined area of the yarn, after the compression is released, to the cutting unit;
cut the predetermined area of the yarn with the cutting unit;
rotate the compression unit and the cutting unit by driving the rotational driving unit;
compress a rear end area of the predetermined area of the yarn with the compression unit and then release the compression;
move the rear end area of the predetermined area of the yarn, after the compression is released, to the cutting unit;
cut the rear end area of the predetermined area of the yarn with the cutting unit; and
rotate the compression unit and the cutting unit by driving the rotational driving unit,
wherein even when forming molded cogs on the yarn, the processing is performed under room temperature conditions without applying heat to the yarn, thereby improving the reliability of the yarn quality.

4. The lifting thread manufacturing apparatus of claim 2,
wherein the processing unit comprises:
at least one pair of the compression units and the cutting units;
at least one pair of processing driving units; and
at least one pair of rotational driving units configured to rotate the at least one pair of the compression units and the cutting units.

5. The lifting thread manufacturing apparatus of claim 4, wherein the at least one pair of the compression units and the cutting units simultaneously form the cogs in areas spaced apart by a predetermined distance on the yarn.

6. The lifting thread manufacturing apparatus of claim 5, wherein the cogs simultaneously formed have inclination angles in opposite directions.

7. The lifting thread manufacturing apparatus of claim 1,
wherein the assembling unit comprises:
a tube mounting unit configured to mount a tube at one end of the lifting thread; and
a cover assembling unit configured to assemble a cover onto the lifting thread with the mounted tube to form a lifting thread assembly.

8. The lifting thread manufacturing apparatus of claim 7, further comprising:
a rotating plate provided with a plurality of lifting thread seating units in which the lifting threads are seated, the rotating plate being configured to rotate; and
an assembling unit conveying unit configured to grip the lifting thread seated in the lifting thread seating unit and detach it from the rotating plate,
wherein the lifting thread manufacturing apparatus is configured to:
mount a tube onto one lifting thread seated in one of the lifting thread seating units using the tube mounting unit;
rotate the rotating plate by a predetermined angle; and
grip the lifting thread with the mounted tube using the assembling unit conveying unit, detach it from the rotating plate, and mount a tube onto another lifting thread seated behind the one lifting thread in the rotational direction of the rotating plate using the tube mounting unit.

9. The lifting thread manufacturing apparatus of claim 8, further comprising an inspection unit configured to perform at least one of:
inspecting the installation state of the lifting thread and the tube; and
inspecting the assembly state of the lifting thread assembly.

10. The lifting thread manufacturing apparatus of claim 1,
wherein the packaging unit comprises:
a first packaging material input unit configured to insert the lifting thread assembly into a first packaging material; and
a second packaging material input unit configured to insert the first packaging material, in which the lifting thread assembly is packaged, into a second packaging material.

11. A lifting thread manufactured according to the lifting thread manufacturing apparatus of any one of claims 1 to 10.

12. A processing unit of a lifting thread manufacturing system, the processing unit being for manufacturing a lifting thread by forming cogs on a yarn, and comprising:
a compression unit configured to compress the yarn;
a cutting unit disposed downstream of the compression unit in the moving direction of the yarn and configured to form the cogs on the yarn;
a processing driving unit configured to control the operation of the compression unit and the cutting unit; and
a rotational driving unit configured to rotate the compression unit, the cutting unit, and the processing driving unit,
wherein, to form the cogs in multiple directions, the processing unit is configured to:
move the yarn to the compression unit;
compress a predetermined area of the yarn with the compression unit and then release the compression;
move the predetermined area of the yarn, after the compression is released, to the cutting unit;
cut the predetermined area of the yarn with the cutting unit;
rotate the compression unit and the cutting unit by driving the rotational driving unit;
compress a rear end area of the predetermined area of the yarn with the compression unit and then release the compression;
move the rear end area of the predetermined area of the yarn, after the compression is released, to the cutting unit;
cut the rear end area of the predetermined area of the yarn with the cutting unit; and
rotate the compression unit and the cutting unit by driving the rotational driving unit,
wherein even when forming molded cogs on the yarn, the processing is performed under room temperature conditions without applying heat to the yarn, thereby improving the reliability of the yarn quality.
